# EUROPEAN PATENT APPLICATION

(11) **EP 3 967 253 A2**
(43) Date of publication of application: **16.03.2022**
(21) Application number: 21195668.5
(22) Date of filing: 09.09.2021
(51) Int. Cl.: A61B 18/14, A61B 18/00, A61B 18/12, A61B 17/00

(54) **FLEX CIRCUIT AND SURFACE MOUNTED ELECTRODE CATHETER**

(30) Priority: 10.09.2020 US 202063076614 P; 08.07.2021 US 202117371000
(71) Applicant: Biosense Webster (Israel) Ltd, Yokneam, 2066717 (IL)
(72) Inventor: BEECKLER, Christopher Thomas, Irvine, 92618 (US); KEYES, Joseph Thomas, Irvine, 92618 (US); GOVARI, Assaf, 2066717 Yokneam (IL)
(74) Representative: Carpmaels & Ransford LLP

(57) **Abstract**

In one embodiment, a medical system includes a catheter to be inserted into a body part, and including an elongated deflectable element including a distal end, a proximal coupler connected to the distal end, an expandable assembly comprising flexible polymer circuit strips, each flexible polymer circuit strip including strip electrodes and a respective contact pad disposed thereon, the flexible polymer circuit strips having respective proximal ends connected to, and disposed circumferentially around, the proximal coupler, and surface mountable electrodes electrically connected to respective ones of the flexible polymer circuit strips, wherein each surface mountable electrode is electrically connected to the respective contact pad of a respective one of the flexible polymer circuit strips using at least one electrically conductive retainer.

## Description

### FIELD OF THE INVENTION

The present invention relates to medical devices, and in particular, but not exclusively to, catheters with electrodes.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the tip electrode(s) of the ablating catheter, and the reference electrode, flowing through the media between the electrodes it, i.e., blood and tissue0. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

US Patent Publication 2014/0276733 of VanScoy, et al., describes an ablation catheter including an elongated body having a proximal end and a distal end. At least one ablation element is disposed on the body between the proximal end and the distal end and configured to ablate renal tissue to control hypertension. At least one localization sensor is disposed on the body and configured to interact with a magnetic field. The at least one localization sensor aids in determining an appropriate target tissue for ablation.

US Patent Publication 2008/0125772 of Stone, et al., describes a catheter and catheter system using energy tailored for remodeling and/or removal of target material along a body lumen, often of atherosclerotic material of a blood vessel of a patient. An elongate flexible catheter body with a radially expandable structure may have a plurality of electrodes or other electrosurgical energy delivery surfaces to radially engage atherosclerotic material when the structure expands. An atherosclerotic material detector system may measure and/or characterize the atherosclerotic material and its location, optionally using impedance monitoring.

US Patent Publication 2011/0137298 of Nguyen, et al., describes an ablation apparatus comprising an ultrasonic transducer which includes a piezoelectric element having a cylindrical shape; a plurality of external electrodes disposed on the outer surface of the piezoelectric element; and at least one internal electrode disposed on the inner surface of the piezoelectric element. The internal electrode(s) provides corresponding internal electrode portions that are disposed opposite the external electrodes with respect to the piezoelectric element, the external electrodes and the at least one internal electrode to be energized to apply an electric field across the piezoelectric element. The ultrasonic ablation zones of the external electrodes are distributed in a staggered configuration so as to span one or more open arc segments around the longitudinal axis, and the ultrasound ablation zones of all external electrodes projected longitudinally onto any lateral plane which is perpendicular to the longitudinal axis span a substantially closed loop around the longitudinal axis.

### SUMMARY

There is provided in accordance with still another embodiment of the present disclosure, a medical system including a catheter configured to be inserted into a body part of a living subject, and including an elongated deflectable element including a distal end, a proximal coupler connected to the distal end, an expandable assembly including a plurality of flexible polymer circuit strips, each flexible polymer circuit strip including multiple strip electrodes and a respective contact pad disposed thereon, the flexible polymer circuit strips having respective proximal ends connected to, and disposed circumferentially around, the proximal coupler, and a plurality of surface mountable electrodes electrically connected to respective ones of the flexible polymer circuit strips, wherein each surface mountable electrode is electrically connected to the respective contact pad of a respective one of the flexible polymer circuit strips using at least one electrically conductive retainer.

Further in accordance with an embodiment of the present disclosure each surface mountable electrode extends around the respective flexible polymer circuit strip.

Still further in accordance with an embodiment of the present disclosure, the system includes an ablation power generator configured to be connected to the catheter, and apply an electrical signal to at least one of the surface mountable electrodes to ablate a tissue of the body part, and a mapping module configured to receive electrical signals from ones of the strip electrodes of the flexible polymer circuit strips, and generate an electro-anatomical map responsively to the received electrical signals.

Additionally, in accordance with an embodiment of the present disclosure the ablation power generator is configured to apply the electrical signal between ones of the surface mountable electrodes.

Moreover, in accordance with an embodiment of the present disclosure the catheter includes a distal coupler, the flexible polymer circuit strips have respective distal ends connected to, and disposed circumferentially around, the distal coupler, the catheter has a distal tip, and the catheter includes a distal electrode disposed at the distal tip of the catheter between the distal ends of the flexible polymer circuit strips.

Further in accordance with an embodiment of the present disclosure, the system includes an ablation power generator configured to be connected to the catheter, and apply an electrical signal between at least one of the surface mountable electrodes and the distal electrode to ablate a tissue of the body part, and a mapping module configured to receive electrical signals from ones of the strip electrodes of the flexible polymer circuit strips, and generate an electro-anatomical map responsively to the received electrical signals.

Still further in accordance with an embodiment of the present disclosure each surface mountable electrode includes a proximal end and a distal end, and the proximal end and the distal end of each surface mountable electrode is electrically connected to the respective contact pad of the respective flexible polymer circuit strip using two respective electrically conductive retainers.

Additionally, in accordance with an embodiment of the present disclosure the proximal end and the distal end of each surface mountable electrode is connected to the respective flexible polymer circuit strip using an adhesive.

Moreover, in accordance with an embodiment of the present disclosure each flexible polymer strip includes multiple layers, a first one of the multiple layers including circuit traces, and a second one of the multiple layers including the strip electrodes and the respective contact pad.

Further in accordance with an embodiment of the present disclosure the catheter includes a pusher including a distal portion, and being configured to be advanced and retracted through the deflectable element, the catheter includes a distal coupler connected to the distal portion of the pusher, and the flexible polymer circuit strips are disposed circumferentially around the distal portion of the pusher, the flexible polymer circuit strips have respective distal ends connected to the distal coupler, and the strips being configured to bow radially outward when the pusher is retracted expanding the expandable assembly from a collapsed form to an expanded form.

Still further in accordance with an embodiment of the present disclosure the surface mountable electrodes are connected to respective ones of the flexible polymer circuit strips in a staggered formation with alternate ones of the surface mountable electrodes being disposed more proximally than other ones of the surface mountable electrodes.

Additionally, in accordance with an embodiment of the present disclosure each of the flexible polymer circuit strips has a respective outer surface, and each of the surface mountable electrodes includes a conductive material biased towards the respective outer surface of a respective one of the flexible polymer circuit strips.

There is also provided in accordance with another embodiment of the present disclosure, a method to manufacture a catheter, including providing a catheter including an elongated deflectable element, a proximal coupler connected to a distal end of the elongated deflectable element, and an expandable assembly including a plurality of flexible polymer circuit strips, each flexible polymer circuit strip including multiple strip electrodes and a respective contact pad disposed thereon, the flexible polymer circuit strips having respective proximal ends connected to, and disposed circumferentially around, the proximal coupler, and electrically connecting a plurality of surface mountable electrodes to respective ones of the flexible polymer circuit strips so that each surface mountable electrode is electrically connected to the respective contact pad of a respective one of the flexible polymer circuit strips using at least one electrically conductive retainer.

Moreover, in accordance with an embodiment of the present disclosure each surface mountable electrode extends around the respective flexible polymer circuit strip.

Further in accordance with an embodiment of the present disclosure the catheter includes a distal coupler, the flexible polymer circuit strips have respective distal ends connected to, and disposed circumferentially around, the distal coupler, the catheter has a distal tip, and the catheter includes a distal electrode disposed at the distal tip of the catheter between the distal ends of the flexible polymer circuit strips.

Additionally, in accordance with an embodiment of the present disclosure the electrically connecting include electrically connecting a proximal end and a distal end of each surface mountable electrode to the respective contact pad of the respective flexible polymer circuit strip using two respective electrically conductive retainers.

Still further in accordance with an embodiment of the present disclosure, the method includes connecting the proximal end and the distal end of each surface mountable electrode to the respective flexible polymer circuit strip using an adhesive.

Additionally, in accordance with an embodiment of the present disclosure, the method includes forming each flexible polymer circuit strip from multiple layers with a first one of the multiple layers including circuit traces and a second one of the multiple layers including the strip electrodes and the respective contact pad.

Moreover in accordance with an embodiment of the present disclosure the catheter includes a pusher including a distal portion, and being configured to be advanced and retracted through the deflectable element, the catheter includes a distal coupler connected to the distal portion of the pusher, and the flexible polymer circuit strips are disposed circumferentially around the distal portion of the pusher, the flexible polymer circuit strips have respective distal ends connected to the distal coupler, and the strips being configured to bow radially outward when the pusher is retracted expanding the expandable assembly from a collapsed form to an expanded form.

Further in accordance with an embodiment of the present disclosure, the method includes connecting the surface mountable electrodes to respective ones of the flexible polymer circuit strips in a staggered formation with alternate ones of the surface mountable electrodes being disposed more proximally that other ones of the surface mountable electrodes.

There is also provided in accordance with still another embodiment of the present disclosure a electrophysiological flexible circuit apparatus, including a resilient substrate extending from a first substrate end to a second substrate end, a flexible circuit strip extending from a first strip end to a second strip end, the flexible circuit strip being coupled to the resilient substrate and having a conductive contact pad disposed on the flexible circuit strip, a plurality of recording electrodes disposed on the flexible circuit strip and each recording electrode being configured to record electrical signals from living tissue, at least one fiber disposed between the resilient substrate and the flexible circuit strip, and an ablation electrode having a hollowed portion extending through the ablation electrode to allow the flexible circuit strip, at least one fiber and the resilient substrate to extend through the hollowed portion, the ablation electrode having at least one electrically conductive retainer that electrically connects the ablation electrode to the conductive contact pad.

Still further in accordance with an embodiment of the present disclosure, the apparatus includes an adhesive that affixes the ablation electrode to any one or more of the following the flexible circuit strips, the resilient substrate, and the conductive contact pad.

Additionally, in accordance with an embodiment of the present disclosure each of the recording electrodes includes a first exposed surface area and each ablation electrode includes a second exposed surface area of at least three times the first exposed surface area.

Moreover, in accordance with an embodiment of the present disclosure the first strip end includes a hinge and a first portion, a second portion, and a third portion, the first portion having a first width that tapers to a second width, the second portion tapering from the second width to a final width in the third portion of the first strip end.

Further in accordance with an embodiment of the present disclosure the at least one fiber includes a yarn.

Still further in accordance with an embodiment of the present disclosure each recording electrode includes a planar electrode.

Additionally, in accordance with an embodiment of the present disclosure the ablation electrode includes an elongated member having a semi-cylindrical cross-section.

### BRIEF DESCRIPTION OF THE DRAWINGS

The present invention will be understood from the following detailed description, taken in conjunction with the drawings in which:
Fig. 1 is a partly pictorial, partly block diagram view of a medical system constructed and operative in accordance with an embodiment of the present invention;
Fig. 2 is a schematic view of a catheter of the system of Fig. 1;
Fig. 3 is a partially exploded view of the catheter of Fig. 2;
Fig. 4A is schematic view of a distal end of the catheter of Fig. 2 without a distal electrode;
Figs. 4B-C are schematic views of a distal end of a flexible polymer circuit strip of the catheter of Fig. 2;
Fig. 5 is a cross-sectional view through line A:A of Fig. 1;
Fig. 6 is a cross-sectional view through line B:B of Fig. 1;
Figs. 7A-B are schematic views illustrating the catheter of Fig. 1 is a deployed form and a collapsed form, respectively;
Fig. 8 is a schematic view of a flexible polymer circuit strip of the catheter of Fig. 2;
Figs. 9 and 10 are schematic view of a flexible polymer circuit strip of the catheter of Fig. 2 showing circuit traces;
Fig. 11 is a schematic view of the flexible polymer circuit strip of Fig. 8 with a surface mountable electrode mounted thereon;
Fig. 12 is a more detailed view of the surface mountable electrode of Fig. 11;
Fig. 13 is a cross-sectional view through line A:A of Fig. 12;
Fig. 14 is a cross-sectional view through line B:B of Fig. 12;
Fig. 15 is a flowchart including steps in a method of manufacturing the catheter of Fig. 2;
Fig. 16 is a schematic view of a biased electrode for use with catheter of Fig. 2; and
Fig. 17 is a cross-sectional view of the biased electrode through line A:A of Fig. 16.

### DESCRIPTION OF EXAMPLE EMBODIMENTS

### OVERVIEW

Diagnostic catheters, such as basket catheters, provide many electrodes to capture electrical signals, such as electrical potentials, from tissue of a body part of a patient. The electrical signals may then be analyzed to provide an indication of the medical state of the body part. For example, the electrical signals may be used to provide an electro-anatomical map. The medical state of the body part may indicate that some of the tissue may require ablation (such as radiofrequency (RF) ablation or irreversible electroporation (IRE)) using a suitable catheter. Although the basket catheter electrodes may be suitable for diagnostic purposes, the electrodes and/or the connections to the electrodes may be too small to be used for ablation. This is particularly true for IRE where high currents are used, and a large electrode surface area is needed to provide effective IRE ablation. Therefore, in such situations, the diagnostic basket catheter is removed from the patient, and a suitable ablation catheter is inserted into the body part of the patient and ablation is performed. Using two catheters increases the length of the medical procedure, may delay essential therapeutic treatment, and may increase trauma of the patient due to the time delay and the use of two catheters.

Embodiments of the present invention solve the above problems by providing a basket catheter which includes both diagnostic electrodes and ablation electrodes thereby providing a combined therapeutic and diagnostic catheter.

The basket catheter includes a distal end expandable assembly made of flexible polymer circuit strips connected together proximally and distally to form a basket. Each strip includes diagnostic electrodes. Each strip also includes an ablation electrode connected to the strip. The ablation electrode of each strip may be slid over that strip, wrapped around that strip, or formed from two parts which are connected together around that strip.

In some embodiments, the ablation electrodes are connected to the flexible polymer circuit strips in a staggered formation with alternate ablation electrodes being disposed more proximally than other ablation electrodes to allow compact stowing of the expandable assembly during insertion of the catheter into the patient and removal of the catheter from the patient.

In some embodiments, each strip includes a contact pad, which is generally formed in the same or similar manner to the diagnostic electrodes. In disclosed embodiments, the contact pad is larger than the diagnostic electrodes. The ablation electrodes are connected to the respective contact pads using a suitable bonding method, such as using solder, conductive epoxy, resistance welding, laser welding, or any other suitable method, to provide an electrical connection between each ablation electrode and the respective contact pad. The ablation electrodes may be connected using one or two bonds. The ablation electrodes are also referred to herein as surface mountable electrodes. The diagnostic electrodes are also referred to herein as strip electrodes. Adhesive may also be used to connect the surface mountable electrodes to the strips in addition to the bonding.

In some embodiments, a mapping module run by a processor receives electrical signals from at least some of the strip electrodes of the flexible polymer circuit strips and generates an electro-anatomical map responsively to the received electrical signals. An ablation power generator is connected to the catheter and applies an electrical signal to one or more of the surface mountable electrodes to ablate (using RF or IRE ablation) the tissue of the body part. In some embodiments, the ablation power generator applies an electrical signal between at least some of the surface mountable electrodes.

In some embodiments, the catheter may include an electrode, referred to herein as a distal electrode, placed at the distal tip of the catheter between the distal ends of the strips. The distal electrode may be used for ablation. In some embodiments, the ablation power generator applies an electrical signal between one or more of the surface mountable electrodes and the distal electrode to ablate (using RF or IRE ablation) the tissue of the body part.

In some embodiments, the ablation electrodes are biased so that there is more material on the tissue contacting side of the strips thereby allowing the distal end assembly to collapse into a small sheath. Another aspect of this electrode design ensures that the bias (i.e., the protrusion) is atraumatic and enhances ablation power on the tissue contacting side. In some embodiments, the atraumatic bias is implemented by smoothing out any sharp edges in the ablation electrodes. Smoothing out sharp edges prevents any damage to tissue or the sheath, and helps prevent high current density that may result in arcing.

### SYSTEM DESCRIPTION

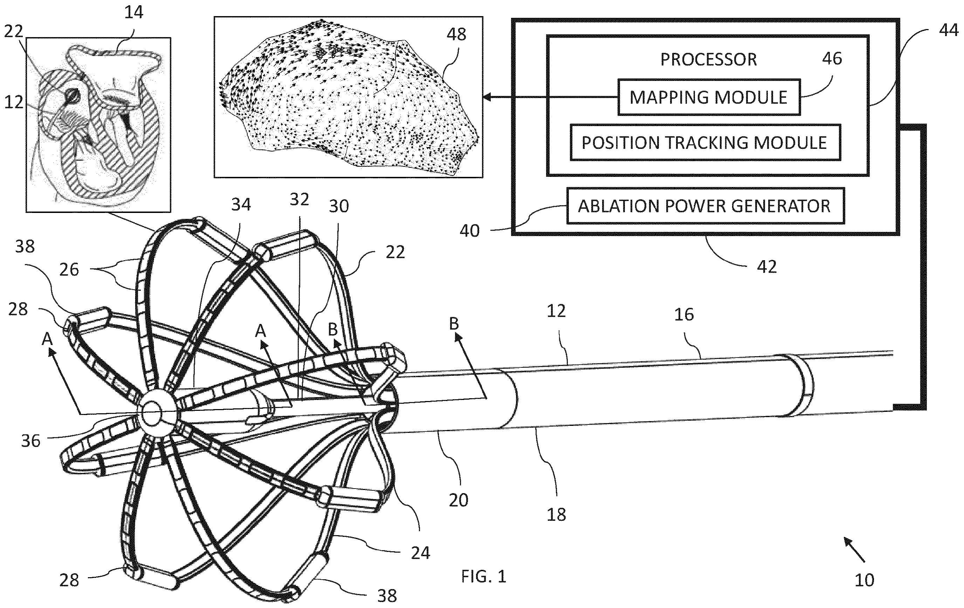

Reference is now made to Fig. 1, which is a partly pictorial, partly block diagram view of a medical system 10 constructed and operative in accordance with an embodiment of the present invention. The medical system 10 includes a catheter 12 configured to be inserted into a body part 14 (e.g., heart chamber) of a living subject.

The catheter 12 includes an elongated deflectable element 16 including a distal end 18. The elongated deflectable element 16 may have any suitable outer diameter and length, for example, the outer diameter may be in a range between 1 mm and 4 mm and the length may be in a range between 1 cm and 15 cm.

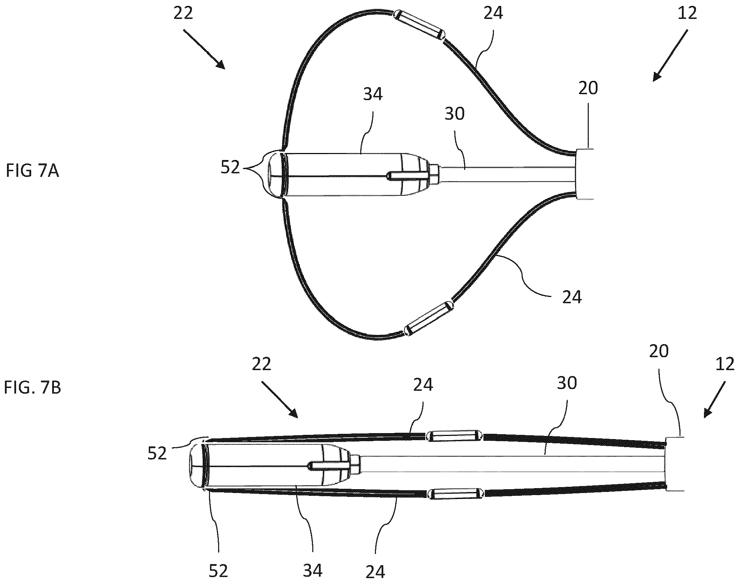

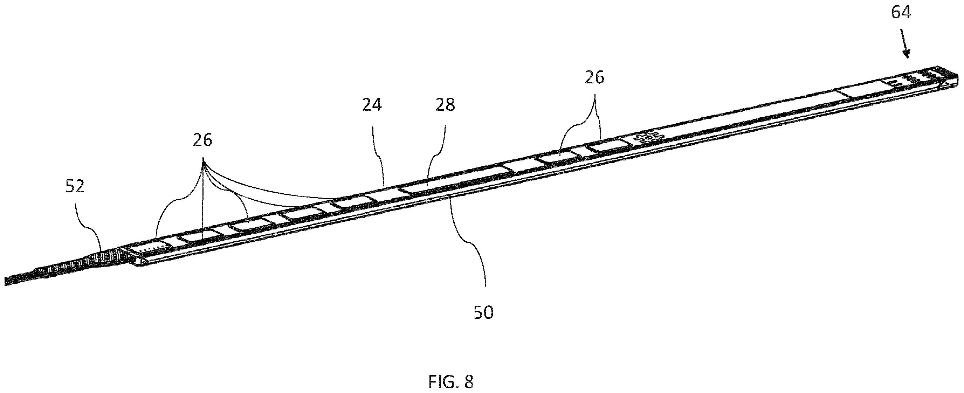

The catheter 12 also includes a proximal coupler 20 connected to the distal end 18. The proximal coupler 20 may be formed as an integral part of the deflectable element 16 or as a separate element, which is then connected to the distal end 18 using any suitable connection method, such as using adhesive, for example, epoxy. The catheter 12 also includes an expandable distal end assembly 22 including flexible circuit strips 24 (only some labeled for the sake of simplicity). In some embodiments, the flexible circuit strips 24 are flexible polymer circuit strips. Each flexible polymer circuit strip 24 includes multiple strip electrodes 26 (e.g., mapping, recording, or diagnostic electrodes) (only some labeled for the sake of simplicity) and a respective conductive contact pad 28 disposed thereon. The contact pads 28 are mostly obscured in Fig. 1 and are shown more clearly in Figs. 8-10, 13, and 14. The catheter 12 includes a pusher 30 including a distal portion 32. The pusher 30 is configured to be advanced and retracted through the deflectable element 16. The catheter 12 also includes a distal coupler 34 connected to the distal portion 32 of the pusher 30. Proximal ends of the flexible polymer circuit strips 24 are connected to the proximal coupler 20, and distal ends of the flexible polymer circuit strips 24 are connected to the distal coupler 34 with the flexible polymer circuit strips 24 being disposed circumferentially around the distal portion 32 of the pusher 30. The flexible polymer circuit strips 24 are configured to bow radially outward when the pusher 30 is retracted expanding the expandable assembly from a collapsed form to an expanded form, described in more detail with reference to Figs. 7A-B.

In some embodiments, the catheter 12 includes a distal electrode 36 disposed at a distal tip of the catheter 12 between the distal ends of the flexible polymer circuit strips 24. In other embodiments, the catheter 12 includes a nose cap (not shown) disposed in the distal coupler 34 instead of the distal electrode 36.

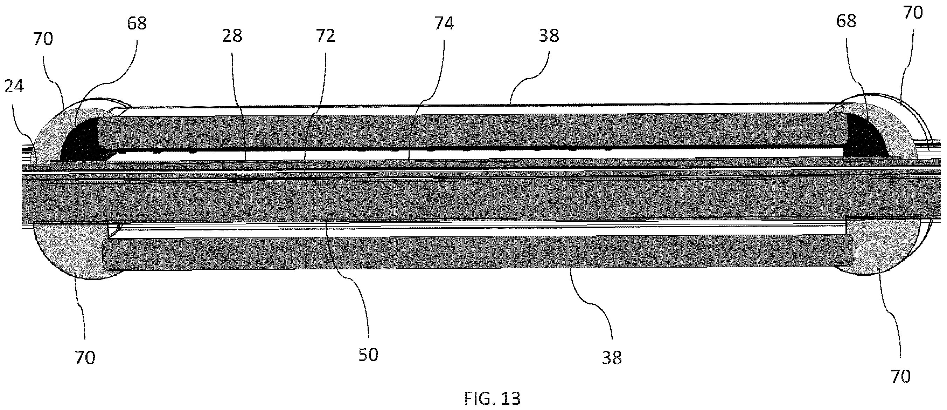

The catheter 12 includes surface mountable electrodes 38 electrically connected to respective one of the flexible polymer circuit strips 24. In some embodiments, one surface mountable electrode 38 is disposed on each flexible polymer circuit strip 24. The surface mountable electrodes 38 are electrically connected to the flexible polymer circuit strips 24 via the contact pads 28, as described in more detail with reference to Fig. 13.

The surface mountable electrodes 38 may be connected at the same position on each flexible polymer circuit strip 24 or in a staggered formation with alternate surface mountable electrodes 38 being disposed more proximally than other surface mountable electrodes 38 so that the expandable assembly 22 may compact efficiently when stowed during insertion into, and removal from the body part 14 of the patient.

In some embodiments, the surface mountable electrodes 38 may be connected to any suitable basket catheter with elements that differ from the elements of the catheter 12.

The medical system 10 includes an ablation power generator 40 configured to be connected to the catheter 12. The ablation power generator 40 may be housed in a console 42. The ablation power generator 40 is configured to apply an electrical signal to one or more of the surface mountable electrodes 38 to ablate tissue of the body part 14. In some embodiments, the ablation power generator 40 is configured to apply an electrical signal between some of the surface mountable electrodes 38 to ablate (using RF or IRE ablation) the tissue. In some embodiments, the ablation power generator 40 is configured to apply an electrical signal between one or more of the surface mountable electrodes 38 and the distal electrode 36 to ablate (using RF or IRE ablation) the tissue of the body part 14.

The medical system 10 includes a processor 44, which is configured to execute a mapping module 46 configured to: receive electrical signals from at least some of the strip electrodes 26 of the flexible polymer circuit strips 24; and generate an electro-anatomical map 48 responsively to the received electrical signals.

In practice, some or all of the functions of the processor 44 may be combined in a single physical component or, alternatively, implemented using multiple physical components. These physical components may comprise hardwired or programmable devices, or a combination of the two. In some embodiments, at least some of the functions of the processor 44 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

The medical system 10 may include other modules and elements which are not shown for the sake of simplicity, such as an electrocardiogram module, a display screen, and user input devices (e.g., keyboard and mouse), by way of example only.

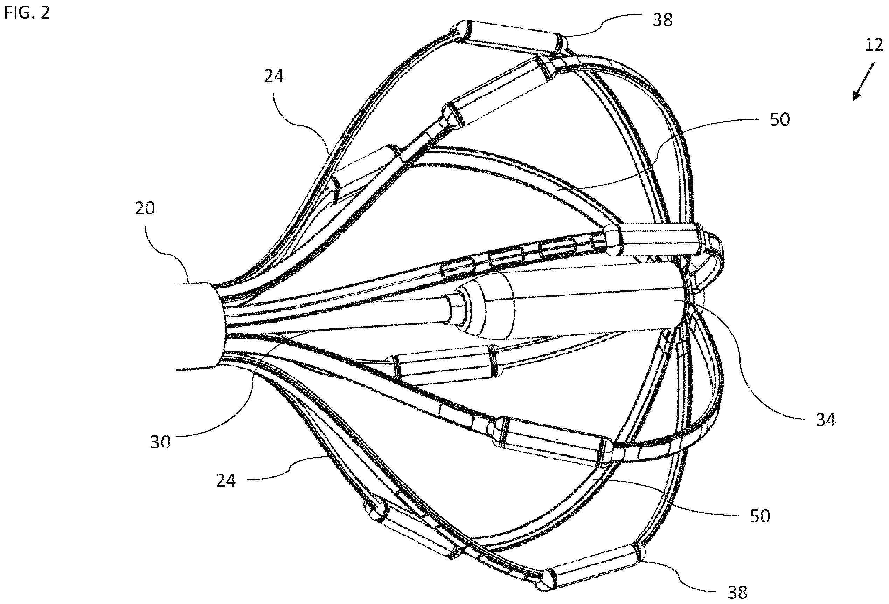

Reference is now made to Fig. 2, which is a schematic view of the catheter 12 of the system 10 of Fig. 1. Fig. 2 shows the proximal coupler 20, the distal coupler 34, the flexible polymer circuit strips 24 (only some labeled for the sake of simplicity), the pusher 30, and the surface mountable electrodes 38 (only some labeled for the sake of simplicity) in more detail.

The inner diameter of the pusher 30 is sized to accommodate wiring. The pusher 30 may be formed from any suitable material, for example, but not limited to polyimide with or without braiding, polyether ether ketone (PEEK) with or without braiding, or polyamide with or without braiding. The distal coupler 34 and the proximal coupler 20 may be formed from any suitable material, for example, but not limited to polycarbonate with or without glass filler, PEEK with or without glass filler, or PEI with or without glass filler.

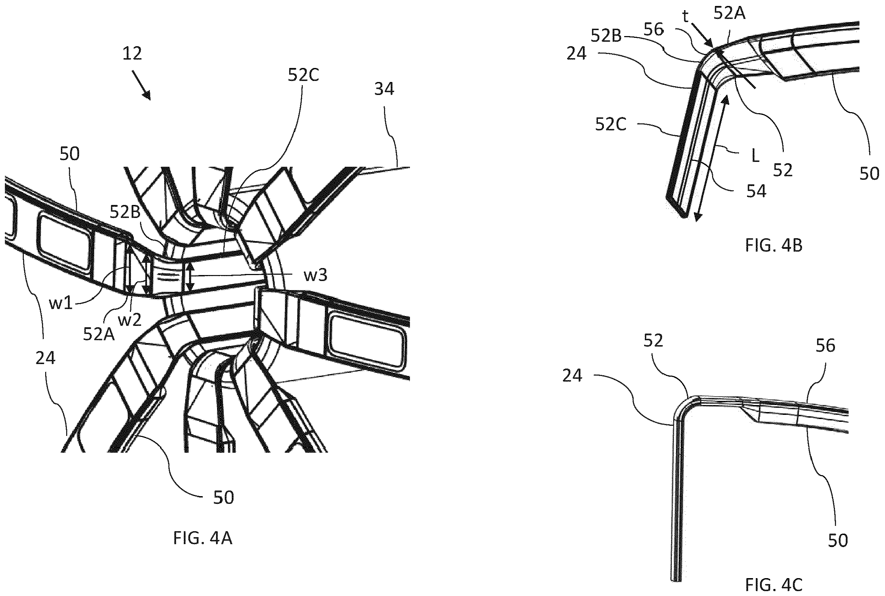

The catheter 12 includes elongated resilient support elements 50 (also referred to herein as resilient substrates) (only some labeled for the sake of simplicity) connected along a given length of respective ones of the flexible polymer circuit strips 24 providing a shape of the expandable assembly 22 in the expanded form of the expandable assembly 22. The elongated resilient support elements 50 may include any suitable material, for example, but not limited to, Nitinol and/or Polyetherimide (PEI). The elongated resilient support elements 50 extend along the inner surface of the respective strips 24 from the proximal coupler 20 until just before the flexible polymer circuit strips 24 enter the distal coupler 34 to allow the flexible polymer circuit strips 24 to bend sufficiently at that point, as described in more detail with reference to Figs. 4A-C. The elongated resilient support elements 50 may have any suitable thickness, for example, in the range of about 0.025 mm to 0.25 mm.

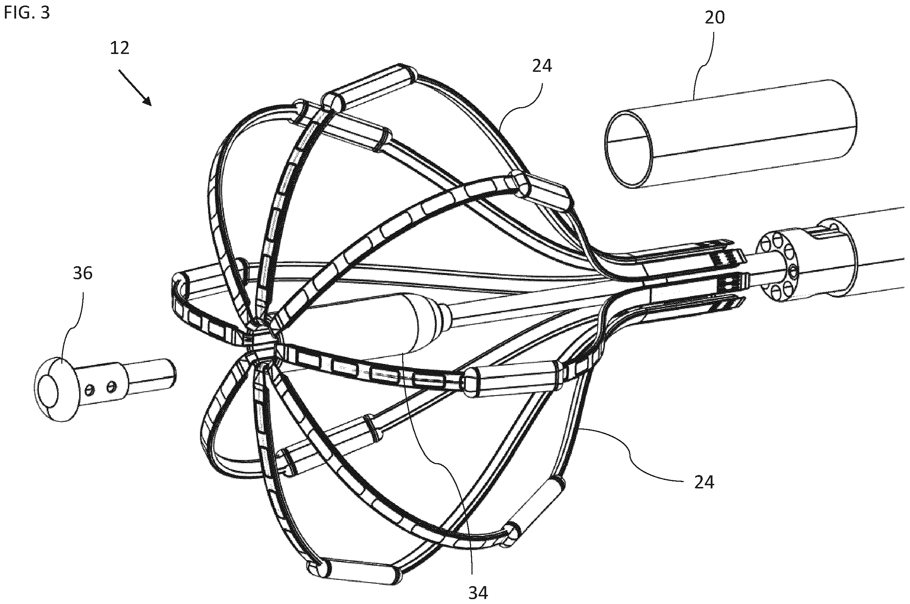

Reference is now made to Fig. 3, which is a partially exploded view of the catheter 12 of Fig. 2. Fig. 3 shows the catheter 12 with the distal electrode 36 (or nose piece) and the proximal coupler 20 removed to illustrate how the flexible polymer circuit strips 24 are connected to the distal coupler 34 and the proximal coupler 20. The proximal ends of the flexible polymer circuit strips 24 are connected to, and disposed circumferentially around, an inner surface of the proximal coupler 20. The distal ends of the flexible polymer circuit strips 24 are connected to, and disposed circumferentially around, an inner surface of the distal coupler 34. The flexible polymer circuit strips 24 may be connected to the proximal coupler 20 and distal coupler 34 using any suitable method, for example, using adhesive (e.g., epoxy) and/or using a pressure fit. The distal ends of the flexible polymer circuit strips 24 are generally bent over and connected to the distal end of the distal coupler 34 to allow the flexible polymer circuit strips 24 to bend at almost 90 degrees to form a flat nose catheter. In some embodiments, the distal ends of the flexible polymer circuit strips 24 may be connected to an outer surface of the distal coupler 34 or together without using a distal coupler.

Reference is now made to Fig. 4A, which is schematic view of a distal end of the catheter 12 of Fig. 2 without the distal electrode 36. Reference is also made to Figs. 4B-C, which are schematic views of a distal end of one of the flexible polymer circuit strips 24 of the catheter 12 of Fig. 2. Fig. 4A shows that the distal ends of the flexible polymer circuit strips 24 bend into the distal coupler 34. In some embodiments, the distal ends of the flexible polymer circuit strips 24 are tapered in order for the flexible polymer circuit strips 24 to fit into the distal coupler 34.

As previously mentioned, the flexible polymer circuit strips 24 are supported using the elongated resilient support elements 50 which extend from the proximal ends of the flexible polymer circuit strips 24 until a hinge section 52 of each flexible polymer circuit strip 24. The hinge section 52 may be reinforced using any suitable material. In some embodiments, the hinge section 52 is reinforced using at least one fiber, such as a yarn 54 (Fig. 4B) that runs from between the flexible polymer circuit strip 24 and the elongated resilient support element 50 until the distal end of the flexible polymer circuit strip 24. The yarn 54 may comprises any one or more of the following: an ultra-high-molecular-weight polyethylene yarn; or a yarn spun from a liquid-crystal polymer. The yarn 54 may be any suitable linear density, for example, in a range between approximately 25 denier and 250 deniers. The flexible polymer circuit strip 24, elongated resilient support element 50, and yarn 54 may be connected together using any suitable method, for example, using an adhesive such as epoxy, and may be covered with a suitable covering 56 (Figs. 4B and 4C), e.g., thermoplastic polyethylene terephthalate (PET) shrink-tubing. Windows are opened in the covering 56 to reveal the strip electrodes 26 and the contact pads 28 (Fig. 1).

It can also be seen in Fig. 4B that the hinge section 52 is much thinner (with thickness "t") than the region including the elongated resilient support element 50. The hinge section 52 may have any suitable thickness, for example, in the range of about 10 to 140 microns. The hinge section 52 has a portion 52A, portion 52B, and an end portion 52C. Portion 52A tapers from a width w1 (Fig. 4A) to a smaller width w2 (Fig. 4A). The portion 52B tapers from the width w2 to a smaller final width w3 (Fig. 4A) in end portion 52C. The width w1 is about 2 times width w3. The length L of the end portion 52C is about 3 mm to ensure that the electrode can be retained in coupler 34 without detaching.

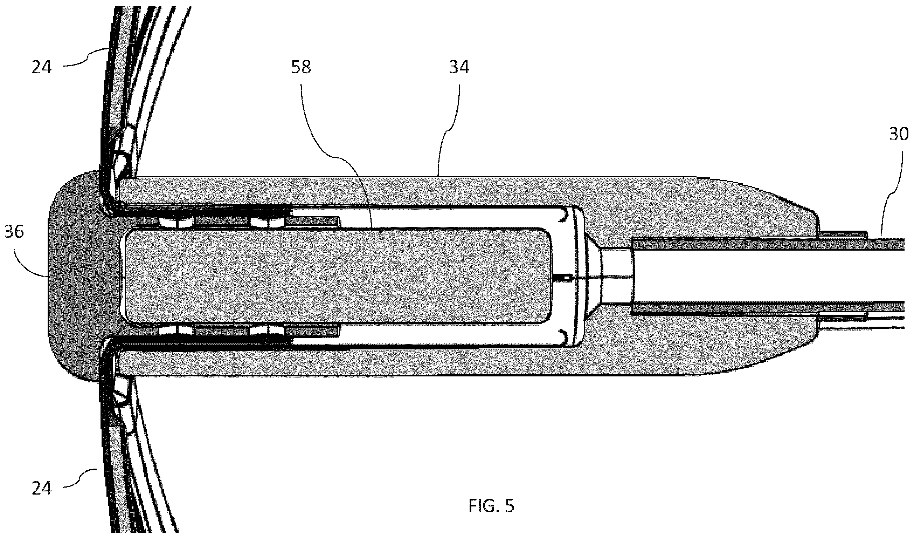

Reference is now made to Fig. 5, which is a cross-sectional view through line A:A of Fig. 1. Fig. 5 illustrates how the distal ends of the flexible polymer circuit strips 24 are connected to the inner surface of the distal coupler 34. A position sensor 58 (such as a magnetic position sensor) is optionally disposed in the distal coupler 34. The distal electrode 36 is inserted into the distal coupler 34 between the flexible polymer circuit strips 24 and the position sensor 58. Fig. 5 illustrates how the distal end of the pusher 30 is connected to the proximal end of the distal coupler 34.

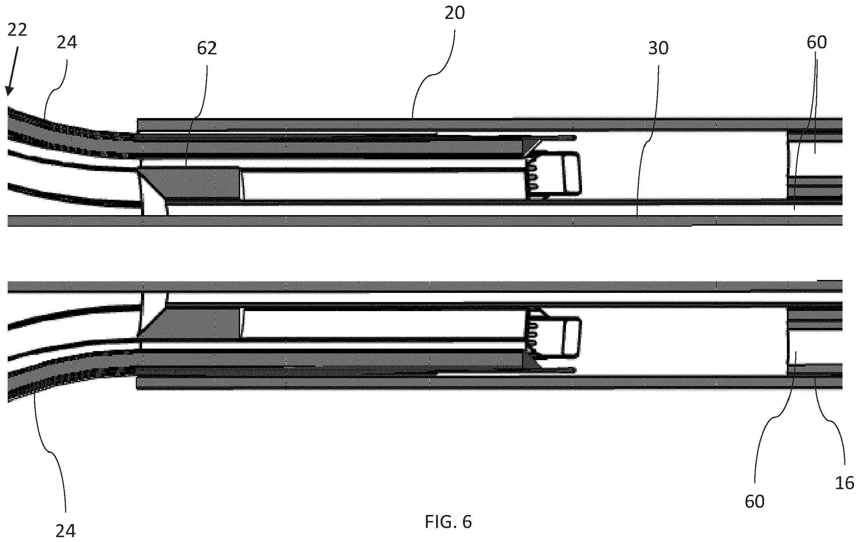

Reference is now made to Fig. 6, which is a cross-sectional view through line B:B of Fig. 1. Fig. 6 illustrates how the proximal ends of the flexible polymer circuit strips 24 are connected to the inner surface of the proximal coupler 20. Fig. 6 also shows that the proximal coupler 20 is connected around an outer surface of the deflectable element 16, which includes lumens 60 to carry wires, and irrigation tubes, for example, from the distal end to the proximal end of the catheter 12. The flexible polymer circuit strips 24 may be additionally held in place using a retaining ring 62. Fig. 6 also shows the pusher 30 extending from one of the lumens 60 of the deflectable element 16 into the expandable assembly 22.

Reference is now made to Figs. 7A-B, which are schematic views illustrating the catheter 12 of Fig. 1 is a deployed form and a collapsed form, respectively. The flexible polymer circuit strips 24 are configured to bow radially outward when the pusher 30 is retracted expanding the expandable assembly 22 from a collapsed form to an expanded form. The collapsed form of the expandable assembly 22 represents the non-stressed form of the flexible polymer circuit strips 24 which are provided with their shape using the elongated resilient support elements 50 (Figs. 4A-C). In some embodiments, the non-stressed form of the expandable assembly 22 is the expanded form. In some embodiments, the expandable assembly 22 collapses when retracted into a sheath (not shown) without needing a pusher or similar element.

In some embodiments, the flexible polymer circuit strips 24 are formed as flat strips. The distal ends of the flexible polymer circuit strips 24 are connected to the inner surface of the distal coupler 34 and then the proximal ends of the flexible polymer circuit strips 24 are connected to the proximal coupler 20 so that in the collapsed form, the angle between a tangent to the distal ends of the flexible polymer circuit strips 24 and an axis of the pusher 30 is close to 180 degrees, while in the expanded form, the angle between the tangent to the distal ends of the flexible polymer circuit strips 24 and the axis is about 90 degrees. Therefore, in operation (when the flexible polymer circuit strips 24 are connected to the distal electrode 36 and the proximal coupler 20) the hinge sections 52 are configured to provide a maximum angular range of movement of the flexible polymer circuit strips 24 of about 90 degrees and generally in excess of 80 degrees. However, the hinge section 52 is capable of bending 180 degrees or more.

Reference is now made to Fig. 8, which is a schematic view of one of the flexible polymer circuit strips 24 of the catheter 12 of Fig. 2. The flexible polymer circuit strip 24 shows the strip electrodes 26 and the contact pad 28. The contact pad 28 is typically formed in the same manner as the strip electrodes 26, except that the contact pad 28 may be longer than each strip electrode 26. In some embodiments, the contact pad 28 may be the same size as the strip electrodes 26. Fig. 8 also shows the hinge section 52 and the elongated resilient support element 50 on the lower side of the flexible polymer circuit strip 24. The flexible polymer circuit strip 24 also includes a contact array 64 for connecting the strip electrodes 26, and the contact pad 28 to wires which extend down the deflectable element 16 (Fig. 1) to the proximal end of the catheter 12.

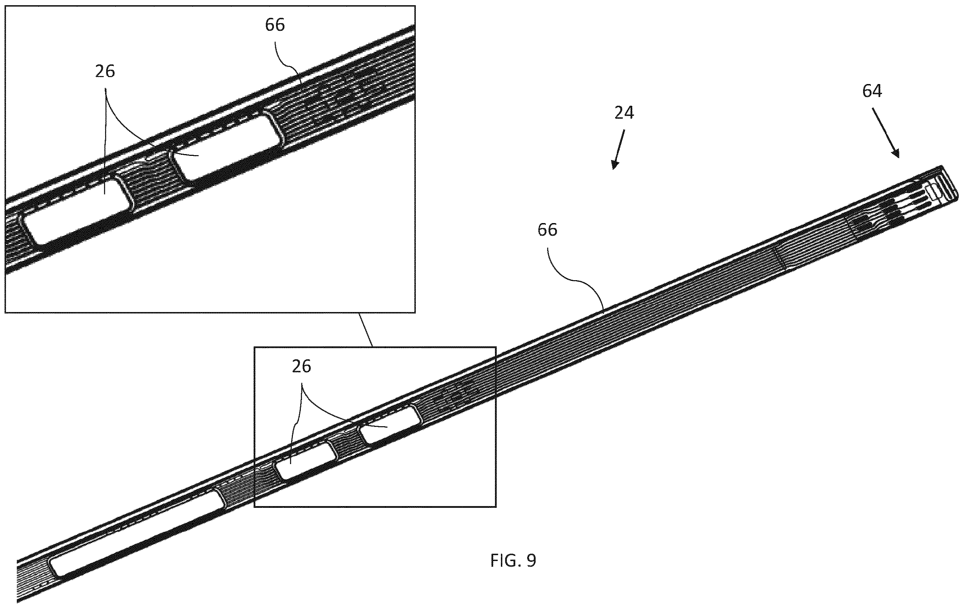

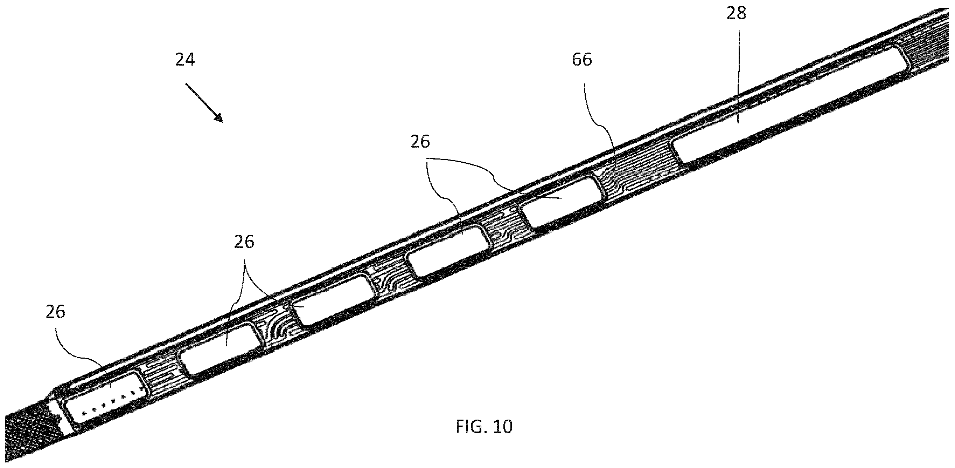

Reference is now made to Figs. 9 and 10, which are schematic view of one of the flexible polymer circuit strips 24 of the catheter 12 of Fig. 2 showing circuit traces 66. The flexible polymer circuit strip 24 of Figs. 9 and 10 are shown in a semi-transparent format to allow visualization of different layers of the flexible polymer circuit strip 24. The flexible polymer circuit strip 24 is generally formed from multiple layers including a lower and upper layer. A lower layer of the flexible polymer circuit strip 24 includes the circuit traces 66 which connect the strip electrodes 26 and the contact pad 28 to the contact array 64. The upper layer includes the strip electrodes 26 and the contact pad 28. The circuit traces 66 in the lower layer are connected to the strip electrodes 26 and the contact pad 28 in the upper layer using vias (not shown). The circuit trace 66 to the contact pad 28 may be wider than the other circuit traces 66 and may be spaced further apart from the other circuit traces 66 to ensure proper isolation. The circuit traces 66 for the strip electrodes 26 may be within the range of about 0.005 mm to 0.1 mm wide (e.g., 0.025 mm) with a spacing in the range of about 0.005 mm to 0.1 mm (e.g., 0.025 mm), while the trace 66 for the contact pad 28 may be in the range of about 0.025 mm to 0.25 mm wide (e.g., 0.125 mm wide) with a spacing to the nearest trace in the range of about 0.010 mm to 0.125 mm (e.g., 0.050 mm). The thickness of the traces 66 may be in the range of about 0.005 mm to 0.100 mm (e.g., 0.010 mm). In some embodiments, the circuit traces 66 may have the same width and spacing. The flexible polymer circuit strips 24 may be composed of any suitable materials. In some embodiments, each of the flexible polymer circuit strips 24 comprises a strip of polyimide. The circuit traces 66 are disposed on the back surface of the strip of polyimide and the strip electrodes 26 and contact pad 28 are disposed on the front surface of the strip of polyimide.

The flexible polymer circuit strips 24 may have any suitable dimensions. For example, the length of the flexible polymer circuit strips 24 may be in the range of 10 mm to 60 mm, e.g., 30 mm, the width of the flexible polymer circuit strips 24 may be in the range of about 0.25 mm to 3 mm, e.g., 0.72 mm, and the thickness of the flexible polymer circuit strips 24 may be in the range of about 0.005 mm to 0.14 mm.

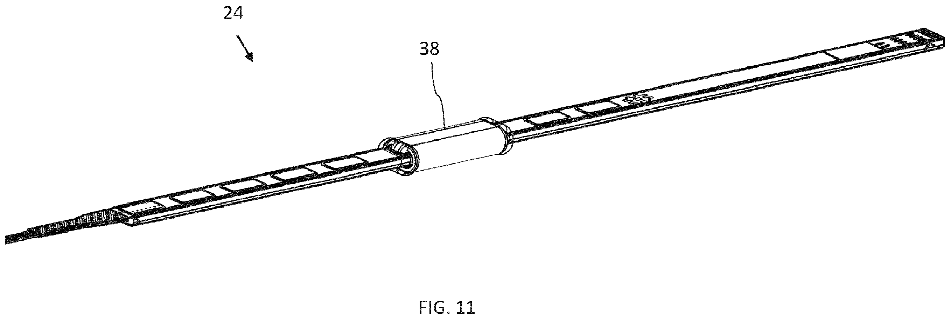

Reference is now made to Fig. 11, which is a schematic view of the flexible polymer circuit strip 24 of Fig. 8 having electrodes 26 (e.g., mapping, recording or diagnostic electrodes) (only some labeled for the sake of simplicity) with one of the surface mountable electrodes 38 mounted thereon. The electrodes 26 are generally used to record electrical signals (e.g., electrocardiogram or intracardiac electrogram signals) generated by living tissue (e.g., cardiac tissue). Each electrode 26 has an exposed surface area A1. Each electrode 26 is typically a planar electrode. The surface mount electrode 38 may be used for ablation, for example, by delivering DC or AC signals via electrode 38. Electrode 38 has an exposed surface area A2 of at least three times the exposed area A1 of each mapping electrode 26. In some embodiments, the exposed surface area A1 is from about 0.08 mm-squared to about Imm-squared. The surface mountable electrode 38 may be formed as a single element which is slid over the flexible polymer circuit strip 24 and the elongated resilient support element 50. In some embodiments, the surface mountable electrode 38 may be formed from two halves which are connected together around strip 24 and the elongated resilient support element 50. In yet other embodiments, the surface mountable electrode 38 may be formed as a band, which is wrapped around the flexible polymer circuit strip 24 and the elongated resilient support element 50. The surface mountable electrodes 38 may be formed from any suitable material, for example, but not limited to gold, gold alloys, platinum, platinum alloys, palladium, or palladium alloys.

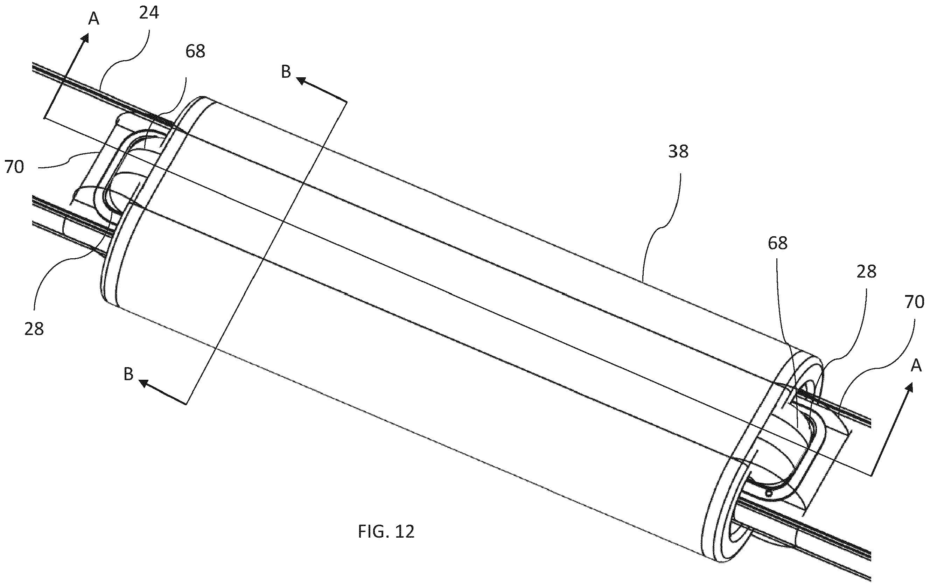

Reference is now made to Fig. 12, which is a more detailed view of the surface mountable electrode 38 of Fig. 11. The surface mountable electrode 38 is shown mounted over the contact pad 28. The surface mountable electrode 38 is electrically connected to the contact pad 28 using two electrically conductive retainers 68 (e.g., using solder, conductive epoxy, resistance welding, laser welding, or any other suitable method). The surface mountable electrode 38 is further secured to the flexible polymer circuit strip 24 (and/or the elongated resilient support elements 50, and/or the contact pad 28) using an adhesive 70, such as a polyurethane adhesive or epoxy. The adhesive 70 may be applied so that the contact pad 28 is electrically isolated from the environment around the catheter 12.

Reference is now made to Fig. 13, which is a cross-sectional view through line A:A of Fig. 12. Fig. 13 shows that the surface mountable electrode 38 extends over the contact pad 28 and below the elongated resilient support element 50. The surface mountable electrode 38 is formed as an elongated member having a semi-cylindrical cross-section as shown in Fig. 13. Fig. 13 also shows two layers of the flexible polymer circuit strip 24, a layer 72 including the circuit traces 66 (shown in each of Figs. 9 and 10) and a layer 74 including the contact pad 28 and the strip electrodes 26 (shown in Fig. 10). The contact pad 28 shown in Fig. 13 is wider than the surface mountable electrode 38. In some embodiments, the contact pad 28 may be narrower or the same width as the surface mountable electrode 38. The surface mountable electrode 38 is electrically connected to the contact pad 28 using at least one electrically conductive retainer 68. In some embodiments, the proximal end and the distal end of the surface mountable electrode 38 is electrically connected to the contact pad 28 using two respective electrically conductive retainers 68. The proximal end and the distal end of the surface mountable electrode 38 is optionally connected to the flexible polymer circuit strips 24 using adhesive 70. The adhesive 70 typically covers the remainder of the contact pad 28 that is not covered by the surface mountable electrode 38. The adhesive 70 may comprise polyurethane adhesive or epoxy, by way of example. While the bonds 68 are typically disposed on the outer surface of the flexible polymer circuit strip 24, the adhesive 70 is generally disposed around the flexible polymer circuit strip 24 and the elongated resilient support element 50 so as to secure the surface mountable electrode 38 to the flexible polymer circuit strip 24 and the elongated resilient support element 50 and prevent liquid contacting the contact pad 28 in use of the catheter 12.

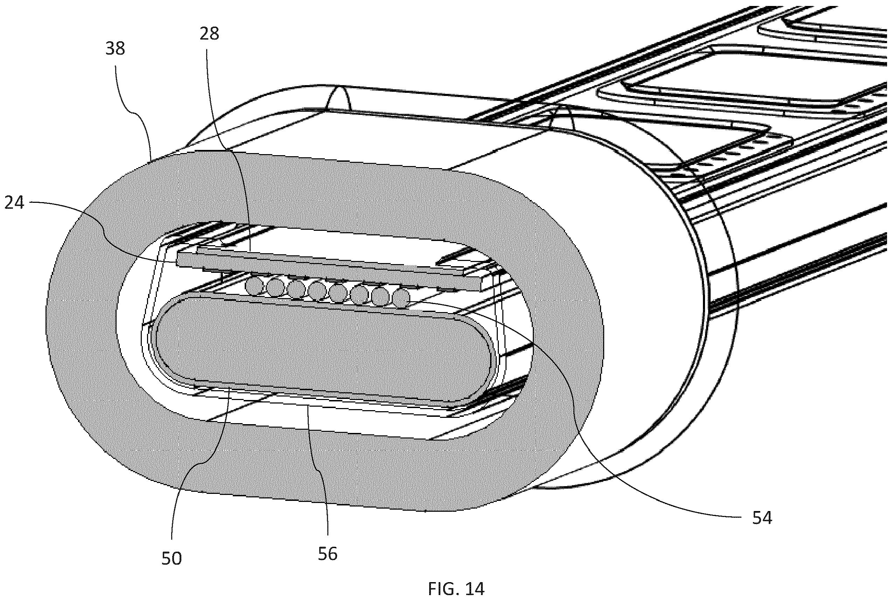

Reference is now made to Fig. 14, which is a cross-sectional view through line B:B of Fig. 12. Fig. 14 shows that the surface mountable electrode 38 extends around the flexible polymer circuit strip 24 and the elongated resilient support element 50. Fig. 14 also shows the yarn 54 (or at least one fiber) sandwiched between the flexible polymer circuit strip 24 and the elongated resilient support element 50. The covering 56 (e.g., shrink-tubing) is shown surrounding the elongated resilient support elements 50 and partially surrounding the flexible polymer circuit strips 24 where a window is opened in the covering 56 to expose the contact pad 28. The surface mountable electrode 38 shown in Fig. 14 is a one-piece hollowed electrode (i.e., an ablation electrode with a hollowed portion extending therethrough) which is slid over the flexible polymer circuit strip 24 and elongated resilient support element 50 combination allowing the flexible polymer circuit strip 24, the yarn 54 (or at least one fiber), and the elongated resilient support elements 50 to extend through the hollowed portion. As previously mentioned, the surface mountable electrode 38 may be formed from two halves which are connected together around strip 24. In yet other embodiments, the surface mountable electrode 38 may be formed as a band, which is wrapped around the flexible polymer circuit strip 24.

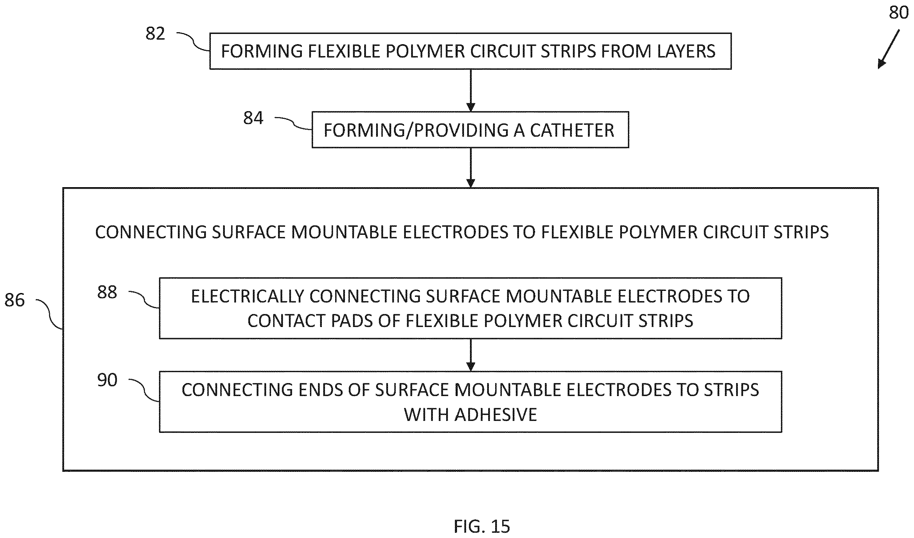

Reference is now made to Fig. 15, which is a flowchart 80 including steps in a method of manufacturing the catheter 12 of Fig. 2. Reference is also made to Fig. 1.

The method includes forming (block 82) each flexible polymer circuit strip 24 from multiple layers, with the layer 72 (Fig. 13) comprising circuit traces 66 (Figs. 9 and 10) and the layer 74 (Fig. 13) including the strip electrodes 26 and the respective contact pad 28.

The method includes forming or providing (block 84) the catheter 12 including: the elongated deflectable element 16; the proximal coupler 20 connected to the distal end 18 of the deflectable element 16; the distal coupler 34; the distal electrode 36; the pusher 30; and the expandable assembly 22. The expandable assembly 22 includes the flexible polymer circuit strips 24. Each flexible polymer circuit strip 24 includes the respective strip electrodes 26 and the respective contact pad 28 disposed thereon. The proximal ends and distal ends of the flexible polymer circuit strips 24 are connected to, and disposed circumferentially around, the proximal coupler 20 and the distal coupler 34, respectively. The distal electrode 36 is disposed at the distal tip of the catheter 12 between the distal ends of the flexible polymer circuit strips 24. The pusher 30 is configured to be advanced and retracted through the deflectable element 16. The distal portion of the pusher 30 is connected to the distal coupler 34. The flexible polymer circuit strips 24 are disposed circumferentially around the distal portion of the pusher 30. The flexible polymer circuit strips 24 are configured to bow radially outward when the pusher 30 is retracted expanding the expandable assembly 22 from a collapsed form to an expanded form.

The method also includes connecting (block 86) the surface mountable electrodes 38 to respective ones of the flexible polymer circuit strips 24. In some embodiments, the method includes connecting the surface mountable electrodes 38 to respective ones of the flexible polymer circuit strips 24 in a staggered formation with alternate ones of the surface mountable electrodes 38 being disposed more proximally that other ones of the surface mountable electrodes 38. The step of block 86 may include the sub-steps of blocks 88 and 90 as follows.

The method may also include electrically connecting (block 88) the surface mountable electrodes 38 to respective ones of the flexible polymer circuit strips 24 so that each surface mountable electrode 38 is electrically connected to the respective contact pad 28 of a respective one of the flexible polymer circuit strips 24 using at least one electrically conductive retainer 68 (Fig. 13), for example, using solder, conductive epoxy, resistance welding, laser welding, or any other suitable method. In some embodiments, the method includes electrically connecting the proximal and distal end of each surface mountable electrode 38 to the respective contact pad 28 of the respective flexible polymer circuit strip 24 using two respective electrically conductive retainers 68.

The method may also include connecting (block 90) the proximal and distal end of each surface mountable electrode 38 to the respective flexible polymer circuit strip 24 using adhesive 70.

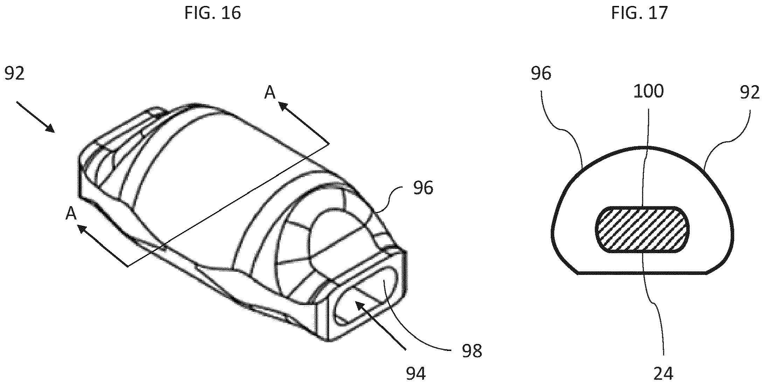

Reference is now made to Fig. 16, which is a schematic view of a biased ablation electrode 92 for use with catheter 12 of Fig. 2. Reference is also made to Fig. 17, which is a cross-sectional view of the biased ablation electrode 92 through line A:A of Fig. 16 with the biased ablation electrode 92 disposed on one of the flexible polymer circuit strips 24.

The biased ablation electrode 92 is a surface mountable electrode and may replace one or more of the surface mountable electrodes 38. The biased ablation electrode 92 is fabricated from a biocompatible electrically conductive material, and has an electrode lumen 94 therethrough. The biased ablation electrode 92 has an outer surface 96 and an inner surface 98 defined by its lumen 94.

Each of the flexible polymer circuit strips 24 has a respective outer surface 100. Each biased ablation electrode 92 comprises a conductive material biased towards the respective outer surface 100 of the respective flexible polymer circuit strip 24.

In some embodiments, for a given biased ablation electrode 92 fitted to a given flexible polymer circuit strip 24, the given flexible polymer circuit strip 24 is planar at the given electrode 92, wherein the plane divides the given electrode 92 asymmetrically so that there is more conductive material on the outer side (of the plane) than on the inner side. In these embodiments, electrodes 92 (i.e., when fitted to flexible polymer circuit strips 24) are biased towards the outer sides of the flexible polymer circuit strips 24, as there is a greater surface area of the given electrode 92 on the outer side compared to the surface area of the given electrode 92 on the inner side.

Examples of materials suitable for forming electrodes 92 include gold, platinum and palladium (and their respective alloys). These materials also have very high thermal conductivity which allows the minimal heat generated on the tissue (i.e., by the ablation energy delivered to the tissue) to be conducted through the electrodes to the back side of the electrodes (i.e., the portions of the electrodes on the inner sides of the flexible polymer circuit strips 24), and then to the blood pool in the heart.

By biasing electrodes 92 to outer surface 100, the electrodes 92 deliver more ablation energy from the outer portion of the electrodes 92 (i.e., significantly more than the ablation energy delivered from the portion of the electrodes on the inner side of the flexible polymer circuit strips 24). The configuration described hereinabove may also enable heat to be transferred from the tissue (i.e., during an ablation procedure) to the portion of the electrodes on the inner side of the flexible polymer circuit strips 24, and the electrodes 92 can be cooled by the blood pool and/or aiming irrigation fluid, e.g., via spray ports (not shown), at the portion of the electrodes 92 on the inner side of the flexible polymer circuit strips 24.

The biased ablation electrodes 92 may be formed using any suitable method, for example, by extruding a non-radially symmetric tube of conductive material. For example, the tube may have a height of 0.04 inches (± 0.003 inches) and a width of 0.048 inches (± 0.003 inches), the lumen may have a height of 0.017 inches (± 0.001 inches) and a width of 0.032 inches (±0.002 inches), the thickness of the conductive material below the lumen may be 0.003 inches (± 0.001 inches), and the thickness of the conductive material above the lumen may be approximately 0.02 inches. In a fabrication step, a cutting instrument may be used to cut a plurality of electrodes 92 from the tube. In some embodiments, each electrode may have a length of approximately 0.08 inches. A manufacturer can use a cutting instrument such as an electrical discharging machining (EDM) wire to fabricate the electrodes from the tube.

The fabrication step may create sharp edges on the electrodes 92. If the catheter 12 is used for performing high-energy ablation procedures such as IRE ablation, applying the high ablation energy to electrodes 92 may result in arcing from the sharp edges. In a smoothing step, the manufacturer smooths out edges on the fabricated electrodes 92. Examples of smoothing processes that the manufacturer can use include, but are not limited to, tumbling/rumbling and barreling.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and sub combinations of the various features described hereinabove, as well as variations and modifications thereof which would occur to persons skilled in the art upon reading the foregoing description and which are not disclosed in the prior art.

## Claims

1. A medical system including a catheter configured to be inserted into a body part of a living subject, and comprising:
an elongated deflectable element including a distal end;
a proximal coupler connected to the distal end;
an expandable assembly comprising a plurality of flexible polymer circuit strips, each flexible polymer circuit strip including multiple strip electrodes and a respective contact pad disposed thereon, the flexible polymer circuit strips having respective proximal ends connected to, and disposed circumferentially around, the proximal coupler; and
a plurality of surface mountable electrodes electrically connected to respective ones of the flexible polymer circuit strips, wherein each surface mountable electrode is electrically connected to the respective contact pad of a respective one of the flexible polymer circuit strips using at least one electrically conductive retainer.

2. The system according to claim 1, further comprising:
an ablation power generator configured to be connected to the catheter, and apply an electrical signal to at least one of the surface mountable electrodes to ablate a tissue of the body part; and
a mapping module configured to: receive electrical signals from ones of the strip electrodes of the flexible polymer circuit strips; and generate an electro-anatomical map responsively to the received electrical signals.

3. The system according to claim 2, wherein the ablation power generator is configured to apply the electrical signal between ones of the surface mountable electrodes.

4. The system according to claim 1, wherein:
each surface mountable electrode includes a proximal end and a distal end; and
the proximal end and the distal end of each surface mountable electrode is electrically connected to the respective contact pad of the respective flexible polymer circuit strip using two respective electrically conductive retainers.

5. The system according to claim 4, wherein the proximal end and the distal end of each surface mountable electrode is connected to the respective flexible polymer circuit strip using an adhesive.

6. The system according to claim 1, wherein each flexible polymer strip includes multiple layers, a first one of the multiple layers comprising circuit traces, and a second one of the multiple layers including the strip electrodes and the respective contact pad.

7. The system according to claim 1, wherein the surface mountable electrodes are connected to respective ones of the flexible polymer circuit strips in a staggered formation with alternate ones of the surface mountable electrodes being disposed more proximally than other ones of the surface mountable electrodes.

8. The system according to claim 1, wherein each of the flexible polymer circuit strips has a respective outer surface, and each of the surface mountable electrodes comprises a conductive material biased towards the respective outer surface of a respective one of the flexible polymer circuit strips.

9. A method to manufacture a catheter, comprising:
providing a catheter including an elongated deflectable element, a proximal coupler connected to a distal end of the elongated deflectable element, and an expandable assembly comprising a plurality of flexible polymer circuit strips, each flexible polymer circuit strip including multiple strip electrodes and a respective contact pad disposed thereon, the flexible polymer circuit strips having respective proximal ends connected to, and disposed circumferentially around, the proximal coupler; and
electrically connecting a plurality of surface mountable electrodes to respective ones of the flexible polymer circuit strips so that each surface mountable electrode is electrically connected to the respective contact pad of a respective one of the flexible polymer circuit strips using at least one electrically conductive retainer.

10. The system according to claim 1 or the method according to claim 9, wherein each surface mountable electrode extends around the respective flexible polymer circuit strip.

11. The system according to claim 1 or the method according to claim 9, wherein:
the catheter includes a distal coupler;
the flexible polymer circuit strips have respective distal ends connected to, and disposed circumferentially around, the distal coupler;
the catheter has a distal tip; and
the catheter includes a distal electrode disposed at the distal tip of the catheter between the distal ends of the flexible polymer circuit strips.

12. The system according to claim 11, further comprising:
an ablation power generator configured to be connected to the catheter, and apply an electrical signal between at least one of the surface mountable electrodes and the distal electrode to ablate a tissue of the body part; and
a mapping module configured to: receive electrical signals from ones of the strip electrodes of the flexible polymer circuit strips; and generate an electro-anatomical map responsively to the received electrical signals.

13. The method according to claim 9, wherein the electrically connecting include electrically connecting a proximal end and a distal end of each surface mountable electrode to the respective contact pad of the respective flexible polymer circuit strip using two respective electrically conductive retainers.

14. The method according to claim 13, further comprising connecting the proximal end and the distal end of each surface mountable electrode to the respective flexible polymer circuit strip using an adhesive.

15. The method according to claim 9, further comprising forming each flexible polymer circuit strip from multiple layers with a first one of the multiple layers comprising circuit traces and a second one of the multiple layers including the strip electrodes and the respective contact pad.

16. The system according to claim 1 or the method according to claim 9, wherein:
the catheter includes a pusher including a distal portion, and being configured to be advanced and retracted through the deflectable element;
the catheter includes a distal coupler connected to the distal portion of the pusher; and
the flexible polymer circuit strips are disposed circumferentially around the distal portion of the pusher;
the flexible polymer circuit strips have respective distal ends connected to the distal coupler; and
the strips being configured to bow radially outward when the pusher is retracted expanding the expandable assembly from a collapsed form to an expanded form.

17. The method according to claim 9, further comprising connecting the surface mountable electrodes to respective ones of the flexible polymer circuit strips in a staggered formation with alternate ones of the surface mountable electrodes being disposed more proximally that other ones of the surface mountable electrodes.

18. An electrophysiological flexible circuit apparatus, comprising:
a resilient substrate extending from a first substrate end to a second substrate end;
a flexible circuit strip extending from a first strip end to a second strip end, the flexible circuit strip being coupled to the resilient substrate and having a conductive contact pad disposed on the flexible circuit strip;
a plurality of recording electrodes disposed on the flexible circuit strip and each recording electrode being configured to record electrical signals from living tissue;
at least one fiber disposed between the resilient substrate and the flexible circuit strip; and
an ablation electrode having a hollowed portion extending through the ablation electrode to allow the flexible circuit strip, at least one fiber and the resilient substrate to extend through the hollowed portion, the ablation electrode having at least one electrically conductive retainer that electrically connects the ablation electrode to the conductive contact pad.

19. The apparatus according to claim 18, further comprising an adhesive that affixes the ablation electrode to any one or more of the following: the flexible circuit strips, the resilient substrate, and the conductive contact pad.

20. The apparatus according to claim 18, wherein each of the recording electrodes comprises a first exposed surface area and each ablation electrode comprises a second exposed surface area of at least three times the first exposed surface area.

21. The apparatus according to claim 18, wherein the first strip end comprises a hinge and a first portion, a second portion, and a third portion, the first portion having a first width that tapers to a second width, the second portion tapering from the second width to a final width in the third portion of the first strip end.

22. The apparatus according to claim 18, wherein the at least one fiber comprises a yarn.

23. The apparatus according to claim 18, wherein each recording electrode comprises a planar electrode.

24. The apparatus according to claim 18, wherein the ablation electrode comprises an elongated member having a semi-cylindrical cross-section.
